# EUROPÄISCHE PATENTANMELDUNG

(11) **EP 2 367 127 A2**
(43) Veröffentlichungstag der Anmeldung: **21.09.2011**
(21) Anmeldenummer: 11157307.7
(22) Anmeldetag: 08.03.2011
(51) Int. Cl.: G06F 19/00, H04L 29/06

(54) **Brückenvorrichtung zur Kopplung eines medizinischen Netzwerks mit einem nicht-medizinischen Netzwerk**

(30) Priorität: 10.03.2010 DE 102010010949
(71) Anmelder: Storz Endoskop Produktions GmbH, 78532 Tuttlingen (DE)
(72) Erfinder: Brüderle, Klaus, 78532 Tuttlingen (DE); Dechow, Dietmar, 78532 Tuttlingen (DE)

(57) **Zusammenfassung**

Eine Brückenvorrichtung (30) zur Kopplung eines medizinischen Netzwerks (10) mit einem nicht-medizinischen Netzwerk (20) umfasst eine Speichereinrichtung (50) zum Speichern von Daten aus dem medizinischen Netzwerk (10), eine erste Schnittstelleneinrichtung (42, 44, 48) zur Kopplung der Speichereinrichtung (50) mit dem medizinischen Netzwerk (10) und eine zweite Schnittstelleneinrichtung (62, 64, 66) zur Kopplung der Speichereinrichtung (50) mit dem nicht-medizinischen Netzwerk (20). Die Brückenvorrichtung (30) ist ausgebildet, um zumindest entweder über die erste Schnittstelleneinrichtung (42, 44, 48) ausschließlich schreibende Zugriffe auf die Speichereinrichtung auszuführen (50) oder über die zweite Schnittstelleneinrichtung (62, 64, 66) ausschließlich lesende Zugriffe auf die Speichereinrichtung (50) auszuführen.

## Beschreibung

Die vorliegende Erfindung bezieht sich auf eine Brückenvorrichtung zur Kopplung eines medizinischen Netzwerks mit einem nicht-medizinischen Netzwerk und auf ein Verfahren zum Bereitstellen von Daten aus einem medizinischen Netzwerk in einem nichtmedizinischen Netzwerk.

Innerhalb einer Klinik, einer ärztlichen Praxis oder einer anderen medizinischen Institution und insbesondere innerhalb eines Operationssaals oder eines anderen medizinischen Behandlungsraums können mehrere medizinische Geräte Daten über ein medizinisches Netzwerk austauschen. Medizinische Geräte sind beispielsweise für die Endoskopie verwendete Geräte, andere Geräte zur bildgebenden oder nicht-bildgebenden Diagnostik, chirurgische Geräte, Herz-Lungen-Maschinen und andere Geräte zur Substitution von Körperfunktionen, Bildschirme und Benutzerschnittstellen für medizinisches Personal. Der Ausfall eines medizinischen Geräts kann eine Gefahr für Gesundheit und Leben eines Patienten darstellen.

Um trotz des vorteilhaften und erwünschten Austauschs von Daten zwischen medizinischen Geräten schädliche Wechselwirkungen zwischen diesen zu vermeiden, wird bei der Entwicklung medizinischer Netzwerke und entsprechender Schnittstellen an den medizinischen Geräten hoher Aufwand betrieben. Um störende Einflüsse von Orten außerhalb des medizinischen Netzwerks zu verhindern, werden medizinische Netzwerke in vielen Fällen als von der IT-Umwelt und insbesondere vom Internet weitgehend oder vollständig isolierte Inseln betrieben.

Eine Isolierung eines medizinischen Netzwerks vom Internet hat aber auch eine Reihe von Nachteilen. Insbesondere ist es wünschenswert, dass medizinisches Personal, das keinen physischen Zugriff auf ein Gerät im medizinischen Netzwerk hat, von medizinischen Geräten gemessene oder an medizinischen Geräten eingestellte Parameter beobachten oder überwachen kann. Vorteilhaft wäre auch eine Auswertung von Vorgängen, die durch Geräte im medizinischen Netzwerk beispielsweise während einer Operation erfasst oder gesteuert werden, um beispielsweise Abläufe zu analysieren und zu optimieren. Auch eine rein technische Überwachung der Geräte im medizinischen Netzwerk wäre vorteilhaft, beispielsweise im Hinblick auf die Einhaltung von Wartungsintervallen und die Planung von Instandhaltungsarbeiten.

Eine Aufgabe der vorliegenden Erfindung besteht darin, eine verbesserte Brückenvorrichtung zur Kopplung eines medizinischen Netzwerks mit einem nicht-medizinischen Netzwerk, ein Verfahren zum Bereitstellen von Daten aus einem medizinischen Netzwerk in einem nicht-medizinischen Netzwerk und ein Computer-Programm mit Programmcode zur Durchführung oder Steuerung eines solchen Verfahrens zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, ein medizinisches Netzwerk und ein nicht-medizinisches Netzwerk über eine Brückenvorrichtung, das eine Speichereinrichtung umfasst, zu verbinden. In der Speichereinrichtung können Daten aus dem medizinischen Netzwerk gespeichert bzw. abgelegt werden, die dann vom nichtmedizinischen Netzwerk aus gelesen werden können. Daten aus dem medizinischen Netzwerk sind auf diese Weise im nicht-medizinischen Netzwerk bzw. für Teilnehmer des nicht-medizinischen Netzwerks verfügbar, ohne dass ein Zugriff vom nicht-medizinischen Netzwerk in das medizinische Netzwerk erforderlich wäre und zugelassen werden müsste. Damit verbindet die vorliegende Erfindung alle Vorteile der Verfügbarkeit von Daten aus dem medizinischen Netzwerk im nicht-medizinischen Netzwerk mit einem hohen Maß an Sicherheit für das medizinische Netzwerk, insbesondere vor Störung und Manipulation aus dem nicht-medizinischen Netzwerk. Die erfindungsgemäße Kopplung des medizinischen Netzwerks mit dem nicht-medizinischen Netzwerk ermöglicht insbesondere eine wirksame Verhinderung eines Denial-of-Service-Angriffs, bei dem durch eine hohe Anzahl an Zugriffen ein Teilnehmer oder ein gesamtes Netzwerk bis hin zu vollständigen Unterbindung von dessen Funktion gestört wird.

Eine Brückenvorrichtung zur Kopplung eines medizinischen Netzwerks mit einem nichtmedizinischen Netzwerk umfasst eine Speichereinrichtung zum Speichern von Daten aus dem medizinischen Netzwerk, eine erste Schnittstelleneinrichtung zur Kopplung der Speichereinrichtung mit dem medizinischen Netzwerk und eine zweite Schnittstelleneinrichtung zur Kopplung der Speichereinrichtung mit dem nicht-medizinischen Netzwerk, wobei die Brückenvorrichtung zumindest entweder ausgebildet ist, um über die erste Schnittstelleneinrichtung ausschließlich schreibende Zugriffe auf die Speichereinrichtung zuzulassen bzw. auszuführen, oder ausgebildet ist, um über die zweite Schnittstelleneinrichtung ausschließlich lesende Zugriffe auf die Speichereinrichtung zuzulassen bzw. auszuführen.

Eine Brückenvorrichtung, wie sie hier beschrieben ist, ist damit insbesondere zumindest entweder ausgebildet, um alle über die erste Schnittstelleneinrichtung eingehende bzw. empfangene lesende Zugriffe auf die Speichereinrichtung zu ignorieren bzw. nicht auszuführen, oder ausgebildet, um alle über die zweite Schnittstelleneinrichtung eingehende bzw. empfangene schreibende Zugriffe auf die Speichereinrichtung zu ignorieren bzw. nicht auszuführen.

Indem vom medizinischen Netzwerk aus ausschließlich schreibende Zugriffe auf die Speichereinrichtung zugelassen oder ausgeführt werden und/oder vom nicht-medizinischen Netzwerk aus ausschließlich lesende Zugriffe auf die Speichereinrichtung ausgeführt oder zugelassen werden, kann eine beabsichtigte oder unbeabsichtigte Störung des medizinischen Netzwerks vom nicht-medizinischen Netzwerk aus verhindert werden. Dies ermöglicht bei einer hohen Sicherheit des medizinischen Netzwerks vor Störungen von außen eine Beobachtung oder Überwachung von Geräten im medizinischen Netzwerk oder ihrer Funktion durch technisches Personal außerhalb der medizinischen Einrichtung, eine Beobachtung von Prozessen, an denen Geräte im medizinischen Netzwerk beteiligt sind, als Grundlage für eine Optimierung dieser Prozesse, eine Verfolgung von Prozessen, an denen Geräte im medizinischen Netzwerk beteiligt sind, durch ausgebildetes oder auszubildendes medizinisches Personal außerhalb der medizinischen Einrichtung, beispielsweise zu Zwecken der Qualitätsüberwachung oder der Schulung, etc.

Bei einer Brückenvorrichtung, wie sie hier beschrieben ist, ist insbesondere zumindest entweder die erste Schnittstelleneinrichtung ausschließlich für schreibende Zugriffe auf die Speichereinrichtung ausgebildet oder die zweite Schnittstelleneinrichtung ausschließlich für lesende Zugriffe auf die Speichereinrichtung ausgebildet. Dazu ist insbesondere Software oder Firmware der Brückenvorrichtung entsprechend ausgebildet. Alternativ kann die erste Schnittstelleneinrichtung und/oder die zweite Schnittstelleneinrichtung physikalisch so ausgebildet sein, dass jegliche Art von Daten lediglich vom medizinischen Netzwerk zur Speichereinrichtung der Brückenvorrichtung bzw. von der Speichereinrichtung zum nicht-medizinischen Netzwerk übertragen werden können.

Die erste Schnittstelleneinrichtung umfasst beispielsweise eine Schreibeinrichtung zum Schreiben von Daten aus dem medizinischen Netzwerk in die Speichereinrichtung und einen Dateneingang zur Kopplung der Schreibeinrichtung mit dem medizinischen Netzwerk. Die Schreibeinrichtung kann Hardware und/oder Software- oder Firmware-Module umfassen. Der Dateneingang umfasst beispielsweise einen Steckverbinder.

Die zweite Schnittstelleneinrichtung umfasst beispielsweise eine Leseeinrichtung zum Lesen von Daten aus der Speichereinrichtung und einen Datenausgang zur Kopplung der Leseeinrichtung mit dem nicht-medizinischen Netzwerk. Die Leseeinrichtung kann Hardware und/oder Software- oder Firmware-Module umfassen. Der Datenausgang umfasst beispielsweise einen Steckverbinder.

Hardware der Schreibeinrichtung kann teilweise oder vollständig mit Hardware der Leseeinrichtung identisch sein. Beispielsweise können eine Speichersteuerung eines Festkörperspeichers oder eines optischen oder magnetischen Massenspeichers sowie Schreib-/ Lese-Köpfe von optischen oder magnetischen Massenspeichern gleichzeitig von der ersten Schnittstelleneinrichtung und der zweiten Schnittstelleneinrichtung genutzt bzw. gleichzeitig Teil der Schreibeinrichtung und Teil der Leseeinrichtung sein. Alternativ sind die Schreibeinrichtung und die Leseeinrichtung vollständig disjunkt, bzw. alle Komponenten der Schreibeinrichtung sind nicht gleichzeitig Komponenten der Leseeinrichtung.

Software- bzw. Firmware-Module der Schreibeinrichtung und der Leseeinrichtung sind insbesondere zumindest teilweise nur entweder der Schreibeinrichtung oder der Leseeinrichtung zugeordnet.

Abhängig von der verwendeten Speichereinrichtung können die Schreibeinrichtung und die Leseeinrichtung hinsichtlich ihrer Hardware-Komponenten und ihrer Software- bzw. Firmware-Module in vielfältiger Weise an die zu erwartenden Datenraten und das erwünschte Sicherheitsniveau angepasst werden.

Eine Brückenvorrichtung, wie sie hier beschrieben ist, kann ausgebildet sein, um in der Speichereinrichtung aktuelle Daten und historische Daten zu speichern und über die zweite Schnittstelleneinrichtung an das nicht-medizinische Netzwerk auszugeben. Aktuelle Daten werden auch als Life-Daten bezeichnet. Mittels der Brückenvorrichtung können somit von einem nicht-medizinischen Netzwerk aus nicht nur jederzeit momentan bzw. zu einem nur wenige Sekunden oder Sekundenbruchteile zurückliegenden Zeitpunkt vorliegende Werte von gemessenen oder gesteuerten Parametern von Geräten im medizinischen Netzwerk abgefragt werden. Zusätzlich können historische Daten, die im medizinischen Netzwerk bzw. an dessen Geräten gar nicht mehr verfügbar sein müssen, aus der Brückenvorrichtung bzw. deren Speichereinrichtung ausgelesen werden. Dies ermöglicht eine retrospektive Analyse der Zustände der Geräte sowie der durch die Geräte im medizinischen Netzwerk beobachteten oder gesteuerten Prozesse. Eine solche Analyse kann Grundlage für eine technische und/oder medizinische Optimierung durch technisches oder medizinisches Personal sein. Aus historischen Daten können ferner Informationen über Verschleißzustände von Geräten im medizinischen Netzwerk abgeleitet werden, die ihrerseits Grundlage für die Planung und Durchführung von Wartungs- und Instandhaltungsarbeiten sein können.

Eine Brückenvorrichtung, wie sie hier beschrieben ist, umfasst insbesondere eine Einrichtung zur Aufbereitung von über die erste Schnittstelleneinrichtung empfangenen Daten, wobei die Einrichtung zumindest entweder zur Bestimmung eines Mittelwerts oder zur Bestimmung eines Zeitintegrals oder zur Bestimmung eines Extremwerts innerhalb eines vorgegebenen Zeitintervalls ausgebildet ist.

Eine derartige Aufbereitung von Daten aus dem medizinischen Netzwerk ermöglicht eine deutliche Reduzierung des Volumens historischer Daten. Dadurch wird das erforderliche Speichervolumen der Speichereinrichtung reduziert. Ferner kann die Bereitstellung historischer Daten vereinfacht und beschleunigt werden.

Die Einrichtung zur Aufbereitung von über die erste Schnittstelleinrichtung empfangenen Daten ist beispielsweise ausgebildet, um für eine Reihe von Zeitintervallen für jeden von dem Gerät erfassten oder gesteuerten Parameter einen Mittelwert, einen Integralwert, einen Maximalwert und einen Minimalwert zu bestimmen bzw. zu berechnen. Die Reihe von Zeitintervallen ist insbesondere in Zeiträumen lückenlos, in denen ein vorbestimmtes Gerät, eine Gruppe von Geräten oder alle Geräte im medizinischen Netzwerk in Betrieb sind. Die Zeitintervalle können für alle Parameter eines Geräts oder für alle Parameter aller Geräte in dem medizinischen Netzwerk gleich sein. Alternativ sind die Zeitintervalle für die Parameter verschiedener Geräte im medizinischen Netzwerk oder für verschiedene Parameter des gleichen Geräts verschieden. Die Längen der Zeitintervalle können fest vorgegeben sein oder beispielsweise abhängig von vorbestimmten Ereignissen eine variable Länge aufweisen. Bei Parametern, die sich selten ändern, können die Zeitintervalle so gewählt sein, dass innerhalb eines Zeitintervalls keine Änderung auftritt. In diesem Fall kann auf die Berechnung von Extremwerten verzichtet werden. Zeitintervalle mit fest vorgegebener Dauer können für Parameter, die sich häufig und/oder schnell ändern können, kurz und für Parameter, die sich selten und/oder langsam ändern, lang gewählt werden.

Bei Parametern, die sich selten ändern und/oder nur eine geringe Anzahl verschiedener Werte annehmen, können alternativ nur die Zeitpunkte, in denen sich die Parameter ändern und die jeweils nach diesen Zeitpunkten vorliegenden Werte ermittelt und erfasst werden. Beispiele für derartige Parameter sind Betriebsmodi von Geräten einschließlich des Ein-oder Ausschaltzustands.

Die von der Einrichtung aufbereiteten historischen Daten, insbesondere Mittelwerte, Extremwerte, Integralwerte, werden zusammen mit Information über den Anfang und das Ende des jeweiligen Zeitintervalls in der Speichereinrichtung abgelegt. Dazu umfasst die Speichereinrichtung insbesondere eine Datenbank oder ist Teil eines Datenbanksystems. Aus dem nicht-medizinischen Netzwerk können jederzeit von der Speichereinrichtung die historischen Daten gelesen werden, die in dem medizinischen Netzwerk zu nahezu jeder beliebigen Zeit in der Vergangenheit vorlagen. Beispielsweise kann für jeden beliebigen interessierenden Zeitpunkt ermittelt werden, von welchem Zeitintervall er umfasst ist. Für dieses Zeitintervall können dann für jeden erfassten und in der Speichereinrichtung abgelegten Parameter der Mittelwert, der Minimalwert und der Maximalwert sowie der Zeitintegralwert seit einem vorbestimmten Bezugs-Zeitpunkt, der vor dem Zeitintervall liegt, aus der Speichereinrichtung ausgelesen und im nicht-medizinischen Netzwerk bereitgestellt werden.

Ein Vorteil des Ablegens bzw. Schreibens von Zeitintegralwerten besteht darin, dass ein Zeitintegralwert über einen längeren Zeitraum mit geringem Aufwand als Differenz der in der Speichereinrichtung abgelegten Zeitintegralwerte am Ende und am Anfang des interessierenden Zeitraums bestimmt werden kann. Parameter wie eine Betriebsstundenzahl eines Geräts, ein gefördertes Volumen oder eine Anzahl von Umdrehungen eines Motors innerhalb eines interessierenden Zeitraums sind auf diese Weise mit geringem Aufwand und schnell bestimmbar.

Die Speichereinrichtung einer Brückenvorrichtung, wie sie hier beschrieben ist, kann einen flüchtigen Speicher und/oder einen nicht-flüchtigen Speicher umfassen. Der flüchtige Speicher ist oder umfasst insbesondere einen mehrfach beschreibbaren Speicher mit wahlfreiem Zugriff (englisch: Random Access Memory = RAM) oder ein anderer Festkörperspeicher. Der nicht-flüchtige Speicher ist oder umfasst insbesondere eine Festplatte, ein anderes magnetisches oder optisches Speichermedium oder ein Festkörperlaufwerk (auch als Halbleiterlaufwerk oder Solid State drive bezeichnet), das beispielsweise einen Flashbasierten Halbleiterspeicher umfasst. Die Brückenvorrichtung ist insbesondere ausgebildet, um im flüchtigen Speicher der Speichereinrichtung aktuelle Daten bzw. von jedem aus dem medizinischen Netzwerk empfangenen Parameter den zuletzt empfangenen Wert zu speichern, und um im nicht-flüchtigen Speicher der Speichereinrichtung historische Daten zu speichern bzw. abzulegen. Insbesondere ist die Brückenvorrichtung ausgebildet, um aktuelle Daten ausschließlich im flüchtigen Speicher der Speichereinrichtung und historische Daten ausschließlich im nicht-flüchtigen Speicher der Speichereinrichtung abzulegen.

Ein Vorteil einer Verwendung einer Speichereinrichtung mit einem flüchtigen Speicher und einem nicht-flüchtigen Speicher kann darin bestehen, dass der flüchtige Speicher in besonders kurzer Zeit und nahezu beliebig oft wiederholt beschrieben werden kann, und dass der nicht-flüchtige Speicher für eine dauerhafte Speicherung historischer Daten auch über Zeiträume ohne Leistungsversorgung hinweg geeignet ist.

Eine Brückenvorrichtung, wie sie hier beschrieben ist, kann ausgebildet sein, um über die erste Schnittstelleneinrichtung empfangene Daten mit einem Zeitstempel zu versehen. Daten von einem Gerät aus dem medizinischen Netzwerk, die bereits von dem Gerät mit einem Zeitstempel versehen sind, können durch die Brückenvorrichtung einen zusätzlichen Zeitstempel erhalten. Alternativ wird der Zeitstempel von dem Gerät durch den Zeitstempel der Brückenvorrichtung ersetzt. Der von der Brückenvorrichtung erzeugte Zeitstempel stellt sicher, dass alle in der Speichereinrichtung der Brückenvorrichtung gespeicherten Daten einer einheitlichen Zeitachse zugeordnet werden können, und verhindert störende Einflüsse von nicht-synchronen Uhren der Geräte im medizinischen Netzwerk.

Eine Brückenvorrichtung, wie sie hier beschrieben ist, kann ausgebildet sein, um Daten einer Operation oder eines anderen medizinischen Prozesses zu archivieren und/oder zu dokumentieren. Dies geschieht insbesondere durch Speichern historischer Daten. Die Brückenvorrichtung kann damit zwei Funktionen gleichzeitig erfüllen, nämlich die Kopplung eines medizinischen Netzwerks mit einem nicht-medizinischen Netzwerk und die Archivierung bzw. Dokumentierung von Daten. Damit kann die Brückenvorrichtung einen Beitrag zur Qualitätssicherung leisten.

Eine Brückenvorrichtung, wie sie hier beschrieben ist, kann ausgebildet sein, um in das medizinische Netzwerk keine Fehlermeldung abzugeben oder um jede Fehlermeldung aus einer vorbestimmten Gruppe von Fehlermeldungen an Empfänger im medizinischen Netzwerk zu unterdrücken. Eine große Anzahl von Fehlermeldungen im medizinischen Netzwerk kann dieses stören, bis hin zu vollständigen Unterbindung der Funktion. Wenn die Brückenvorrichtung beabsichtigt oder unbeabsichtigt vom nichtmedizinischen Netzwerk aus veranlasst werden kann, dass sie Fehlermeldungen in das medizinische Netzwerk sendet, kann dies ein Sicherheitsrisiko für das medizinische Netzwerk darstellen. Wenn hingegen die Brückenvorrichtung ausgebildet ist, um überhaupt keine Fehlermeldungen oder nur Fehlermeldungen aus einer vorbestimmten Gruppe von Fehlermeldungen in das medizinische Netzwerk zu senden, kann dieses Risiko ausgeschlossen oder zumindest gemindert werden.

Ein Verfahren zum Bereitstellen von Daten aus einem medizinischen Netzwerk in einem nicht-medizinischen Netzwerk umfasst folgende Schritte:
Koppeln einer Brückenvorrichtung über eine erste Schnittstelleneinrichtung der Brückenvorrichtung mit dem medizinischen Netzwerk;
Koppeln der Brückenvorrichtung über eine zweite Schnittstelleneinrichtung der Brückenvorrichtung mit dem nicht-medizinischen Netzwerk;
Empfangen von Daten aus dem medizinischen Netzwerk über die erste Schnittstelleneinrichtung;
Schreiben der empfangenen Daten an einen Speicherort einer Speichereinrichtung der Brückenvorrichtung;
Lesen von Daten von der Speichereinrichtung;
Bereitstellen der von der Speichereinrichtung gelesenen Daten an der zweien Schnittstelle.

Indem vom medizinischen Netzwerk empfangene Daten in einer Speichereinrichtung einer Brückenvorrichtung abgelegt und später ausgelesen und dem nicht-medizinischen Netzwerk bereitgestellt werden, kann wie bei der oben beschriebenen Brückenvorrichtung ein hohes Maß an Sicherheit mit den Vorteilen einer Kopplung des medizinischen Netzwerks mit dem nicht-medizinischen Netzwerk verbunden werden. Insbesondere können beabsichtigte oder unbeabsichtigte Störungen des medizinischen Netzwerks aus dem nichtmedizinischen Netzwerk wirksam unterdrückt werden.

Ein Verfahren, wie es hier beschrieben ist, kann ferner zumindest einen der folgenden Schritte umfassen:
Auswählen von Daten aus dem medizinischen Netzwerk, die in die Speichereinrichtung zu schreiben sind, an einer Benutzerschnittstelle im medizinischen Netzwerk;
Empfangen einer Leseanweisung über die zweite Schnittstelle;
Verwerfen einer über die erste Schnittstelleneinrichtung empfangenen Leseanweisung;
Verwerfen einer über die zweite Schnittstelleneinrichtung empfangenen Schreibanweisung;
Prüfen einer über die zweite Schnittstelleneinrichtung empfangenen Anweisung und Verwerfen der Anweisung, wenn diese keine Leseanweisung ist;
Aufbereiten historischer Daten;
Schreiben historischer Daten an einen Speicherort der Speichereinrichtung.

Insbesondere sieht das Verfahren das Auswählen von zu schreibenden Daten an einer Benutzerschnittstelle außerhalb des medizinischen Netzwerks nicht vor.

Die vorliegende Erfindung ist als Verfahren oder als Computer-Programm mit Programmcode zur Durchführung oder Steuerung eines solchen Verfahrens, wenn das Computer-Programm auf einem Computer oder einem Prozessor abläuft, implementierbar. Ferner ist die Erfindung als Computer-Programm-Produkt mit auf einem maschinenlesbaren Träger (beispielsweise einem ROM-, PROM-, EPROM-, EEPROM- oder Flash-Speicher, einer CD-ROM, DVD, HD-DVD, Blue-ray Disk, Diskette oder Festplatte) oder in Form von Firmware gespeichertem Programmcode zur Durchführung von einem der genannten Verfahren, wenn das Computer-Programm-Produkt auf einem Computer, Rechner oder Prozessor abläuft, implementierbar. Ferner kann die vorliegende Erfindung als digitales Speichermedium (beispielsweise ROM-, PROM-, EPROM-, EEPROM- oder Flash-Speicher, CD-ROM, DVD, HD-DVD, Blu-ray Disk, Diskette oder Festplatte) mit elektronisch auslesbaren Steuersignalen, die so mit einem programmierbaren Computer- oder Prozessor-System zusammenwirken können, dass eines der beschriebenen Verfahren ausgeführt wird, implementiert werden.

Ferner kann die vorliegende Erfindung als Steuerung implementiert werden, wobei die Steuerung ausgebildet ist, um eines der beschriebenen Verfahren auszuführen, oder wobei die Steuerung ein Computer-Programm, ein Computer-Programm-Produkt oder ein digitales Speichermedium umfasst, wie sie im vorangehenden Absatz beschrieben wurden.

### Kurzbeschreibung der Figuren

Nachfolgend werden Ausführungsformen anhand der beigefügten Figuren näher erläutert. Es zeigen:
- Figur 1: eine schematische Darstellung einer Brückenvorrichtung;
- Figur 2: eine schematische Darstellung einer weiteren Brückenvorrichtung;
- Figur 3: ein schematisches Flussdiagramm eines Verfahrens zum Bereitstellen von Daten aus einem medizinischen Netzwerk in einem nicht-medizinischen Netzwerk.

### Beschreibung der Ausführungsformen

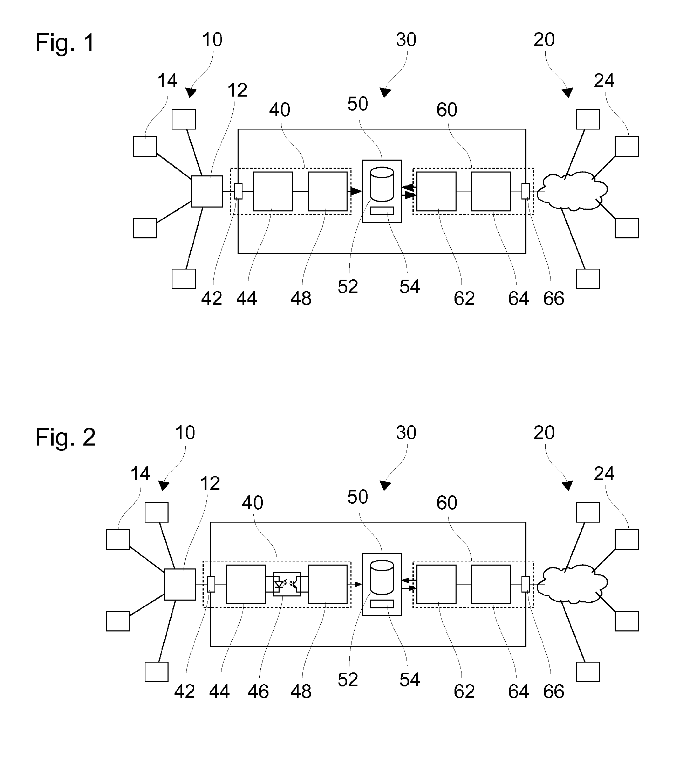

Figur 1 zeigt eine schematische Darstellung eines medizinischen Netzwerks 10, eines nicht-medizinischen Netzwerks 20 und einer Brückenvorrichtung 30, die das medizinische Netzwerk 10 mit dem nicht-medizinischen Netzwerk 20 koppelt. Die Brückenvorrichtung wird auch als Geräteüberwachungs-Serviceprovider bzw. Device Monitor Service Provider bezeichnet.

Das medizinische Netzwerk 10 umfasst einen oder mehrere Knoten 12, mit dem bzw. mit denen ein oder mehrere Geräte 14 über elektrische oder optische Signalkabel oder mittels elektromagnetischer oder optischer Signale, die durch den freien Raum übertragen werden, gekoppelt ist. Das medizinische Netzwerk 10 kann anstelle der in Figur 1 angedeuteten sternförmigen Topologie eine Bus-Topologie oder eine beliebige andere Topologie aufweisen. Beispielsweise für die Geräte 14 sind Kamerasteuerungen oder Kameras, Lichtquellen, Steuerungen für motorische Antriebe und andere Geräte für diagnostische, chirurgische und/oder therapeutische Zwecke. Das medizinische Netzwerk ist beispielsweise ein Storz Communication Bus (SCB) entsprechend dem als OR1 bezeichneten Konzept von Karl Storz.

Das nicht-medizinische Netzwerk 20 umfasst Geräte 24, beispielsweise Arbeitsplatzrechner, und weist eine beliebige Topologie auf. Das nicht-medizinische Netzwerk 20 kann ein lokales Netzwerk, das Internet oder Teile des Internets umfassen.

Die Brückenvorrichtung 30 umfasst einen medizinischen Bereich 40, eine Speichereinrichtung 50 und einen nicht-medizinischen Bereich 60. Die Brückenvorrichtung 30 kann Bereiche, Komponenten, Funktionseinheiten und Hardware- und Software-Module umfassen, die in Figur 1 nicht gezeigt und nachfolgend nicht näher beschrieben sind. Dazu zählen beispielsweise eine Leistungsversorgungseinrichtung für die gesamte Brückenvorrichtung oder mehrere Leistungsversorgungseinrichtungen für verschiedene Bestandteile, Bereiche oder Komponenten der Brückenvorrichtung 30.

Die nachfolgend beschriebenen Komponenten der Brückenvorrichtung 30 können weitere Einheiten umfassen oder zu größeren Komponenten oder Einheiten zusammengefasst sein.

Die nachfolgend beschriebenen Funktionen der Brückenvorrichtung 30 können in einer anderen Weise auf die Komponenten und Module der Brückenvorrichtung 30 aufgeteilt bzw. von den Komponenten realisiert sein als es nachfolgend anhand der Figur 1 dargestellt ist.

Der medizinische Bereich 40 der Brückenvorrichtung 30 umfasst einen Dateneingang 42 zur Kopplung mit dem medizinischen Netzwerk 10. Der Dateneingang 42 umfasst beispielsweise einen Steckverbinder zur Aufnahme eines Endes eines optischen oder elektrischen Signalkabels zur Verbindung mit dem medizinischen Netzwerk 10, insbesondere mit dem Knoten 12. Alternativ umfasst der Dateneingang 42 eine Empfangseinrichtung zum Empfangen oder eine Sende- und Empfangseinrichtung zum Senden und Empfangen von elektromagnetischen oder Licht-Signalen im infraroten.

Der medizinische Bereich 40 der Brückenvorrichtung 30 umfasst ferner eine Datenempfangseinrichtung 44 mit einem Eingang, der mit einem Ausgang des Dateneingangs 42 gekoppelt ist. Die Datenempfangseinrichtung 44 ist ausgebildet, um über den Dateneingang 42 Daten aus dem medizinischen Netzwerk 10 zu empfangen. Die von der Datenempfangseinrichtung empfangenen Daten umfassen insbesondere aktuelle Werte von Parametern, die an Geräten 14 im medizinischen Netzwerk 10 einstellbar sind oder von Geräten 14 im medizinischen Netzwerk 10 gemessene oder gesteuert werden.

Die Datenempfangseinrichtung 44 kann ausgebildet sein, um bei Geräten 14 im medizinischen Netzwerk 10 Parameter zu abonnieren. Die Geräte 14 senden die aktuellen Werte abonnierter Parameter bei jeder Veränderung des Parameters, bei jeder Veränderung des Parameters, die über einer vorbestimmten positiven Veränderungsschwelle oder unter einer vorbestimmten negativen Veränderungsschwelle liegt, bei Vorliegen einer anderen vorbestimmten Bedingung und/oder zu vorbestimmten Zeitpunkten oder in vorbestimmten Zeitabständen. Die Brückenvorrichtung 30 weist insbesondere zumindest einen Betriebsmodus auf, in dem die Kommunikation zwischen der Brückenvorrichtung 30 und einem Gerät 14 über den Dateneingang 42 zugelassen wird, die zum Abonnieren von Parametern erforderlich ist. Ein ähnlicher Weg des Datenbezugs wird in anderen Gebieten der Informationstechnologie auch als Subscribing bezeichnet.

Alternativ oder zusätzlich kann die Datenempfangseinrichtung 44 ausgebildet sein, um die Werte vorbestimmter Parameter zu vorbestimmten Zeitpunkten oder in vorbestimmten Zeitabständen oder bei Vorliegen vorbestimmter Bedingungen von Geräten 14 im medizinischen Netzwerk 10 abzufragen. In Anlehnung an den Sprachgebrauch in anderen Bereichen der Informationstechnologie wird dieser Weg des Datenbezugs im Folgenden auch als Polling bezeichnet. Abhängig von der Art des medizinischen Netzwerks 10 und seinen Eigenschaften, insbesondere Protokoll und Bandbreite, kann ein Verzicht auf die zuletzt beschriebene Polling-Funktionalität einen Sicherheitsgewinn darstellen, weil eine beabsichtigte oder unbeabsichtigte Belastung des medizinischen Netzwerks 10 und seiner Bandbreite durch häufiges Polling verhindert werden kann.

Die Datenempfangseinrichtung 44 kann ausgebildet sein, damit ein Benutzer über eine Benutzerschnittstelle Daten bzw. Parameter auswählen kann, die über den Dateneingang 42 aus dem medizinischen Netzwerk 10, beispielsweise durch Subscribing oder Polling, zu empfangen sind. Die Benutzerschnittstelle umfasst beispielsweise einen Bildschirm, eine Tastatur und/oder eine Computermaus. Die Benutzerschnittstelle kann unmittelbar oder über das medizinische Netzwerk 10 mit der Brückenvorrichtung 30 gekoppelt sein. Wenn die Benutzerschnittstelle über das medizinische Netzwerk 10 mit der Brückenvorrichtung 30 gekoppelt ist, muss die Datenempfangseinrichtung 44 zumindest einen Betriebsmodus aufweisen, in dem die für den Auswahlprozess erforderliche Kommunikation der Brückenvorrichtung 30 mit der Benutzerschnittstelle über den Dateneingang 42 zugelassen wird.

Die Datenempfangseinrichtung 44 der Brückenvorrichtung 30 kann ferner ausgebildet sein, um jede Ausgabe bzw. jedes Senden von Daten, Befehlen, Anweisungen oder anderer Information über den Dateneingang 42 in das medizinische Netzwerk 10 zu unterdrücken. Die beschriebene Polling-Funktionalität ist in diesem Fall nicht realisierbar. Alternativ kann die Datenempfangseinrichtung 44 ausgebildet sein, um lediglich Befehle oder Anweisungen aus einer vorbestimmten Gruppe von Befehlen bzw. Anweisungen über den Dateneingang 42 in das medizinische Netzwerk 10 zu senden. Ferner kann die Datenempfangseinrichtung 44 ausgebildet sein, um in vorbestimmten Zeitintervallen höchstens eine vorbestimmte maximale Anzahl von Befehlen bzw. Anweisungen über den Dateneingang 42 in das medizinische Netzwerk 10 zu senden. Abhängig von den Eigenschaften des medizinischen Netzwerks 10 kann die höchste Sicherheit gegen beabsichtigte oder unbeabsichtigte Störungen des medizinischen Netzwerks 10 durch die Brückenvorrichtung 30 in vielen Fällen dadurch erzielt werden, dass die Datenempfangseinrichtung 44 ausgebildet ist, um keinerlei Befehle oder Anweisungen über den Dateneingang 42 in das medizinische Netzwerk 10 zu senden.

Die Datenempfangseinrichtung 44 kann ferner ausgebildet sein, um über den Dateneingang 42 lediglich Daten zu empfangen. Beispielsweise ist die Datenempfangseinrichtung 44 ausgebildet, um über den Dateneingang 42 aus dem medizinischen Netzwerk 10 empfangene Befehle und Anweisungen oder alle Befehle und Anweisungen aus einer vorbestimmten Gruppe von Befehlen bzw. Anweisungen zu ignorieren. Insbesondere ist die Datenempfangseinrichtung 44 ausgebildet, um über den Dateneingang 42 aus dem medizinischen Netzwerk 10 empfangene Leseanweisungen zu ignorieren. Anstatt Anweisungen zu ignorieren bzw. nicht auszuführen, kann die Datenempfangseinrichtung 44 ausgebildet sein, um auf Anweisungen bzw. Befehle in vorbestimmter, sicherer Weise zu antworten. Eine sichere Antwort umfasst beispielsweise eine kurze, von der Anweisung bzw. dem Befehl unabhängige Nachricht, die beim Kommunikationspartner keine weitere Aktion auslöst.

Insbesondere ist die Datenempfangseinrichtung 44 ausgebildet, um jede über den Dateneingang 42 aus dem medizinischen Netzwerk 10 empfangene Anweisung darauf zu prüfen, ob es sich um eine Leseanweisung handelt, und um die Anweisung zu verwerfen bzw. zu ignorieren oder auf eine sichere Weise zu beantworten, wenn es sich um eine Leseanweisung handelt.

Der medizinische Bereich 40 der Brückenvorrichtung 30 umfasst ferner eine Schreibeinrichtung 48 zum Schreiben bzw. Ablegen von Daten an Speicherorten der Speichereinrichtung 50. Ein Dateneingang der Schreibeinrichtung 48 ist mit einem Datenausgang der Datenempfangseinrichtung 44 gekoppelt, und ein Datenausgang der Schreibeinrichtung 48 ist mit einem Dateneingang der Speichereinrichtung 50 gekoppelt.

Die Schreibeinrichtung 48 kann ausgebildet sein, um von der Datenempfangeinrichtung 44 empfangene Daten mit einem Zeitstempel zu versehen, der von der Schreibeinrichtung 48 erzeugt oder von einer in Figur 1 nicht dargestellten Einrichtung empfangen wurde, und mit diesem Zeitstempel in die Speichereinrichtung 50 zu schreiben.

Ferner kann die Schreibeinrichtung 48 ausgebildet sein, um sowohl aktuelle als auch historische Daten in der Speichereinrichtung 50 zu schreiben. Aktuelle Daten sind die jeweils jüngsten über den Dateneingang 42 und die Datenempfangseinrichtung 44 empfangenen Werte von Parametern. Historische Daten sind zu vorangehenden Zeitpunkten vorliegende Werte der Parameter oder aus diesen Werten berechnete Größen. Die Schreibeinrichtung 48 ist insbesondere ausgebildet, um für Zeitintervalle vorgegebener oder variabler Länge Mittelwerte, Minimalwerte und/oder Maximalwerte von Parametern zu bestimmen bzw. zu berechen.

Ferner kann die Schreibeinrichtung 48 ausgebildet sein, um jeweils für das Ende eines solchen Zeitintervalls oder für einen anderen Zeitpunkt Zeitintegrale von Parametern für einen Zeitraum von einem vorbestimmten Referenz-Zeitpunkt in der Vergangenheit (beispielsweise Herstellung oder Inbetriebnahme eines Geräts 14) bis zu dem Zeitpunkt zu bestimmen bzw. zu berechnen. Die Schreibeinrichtung 48 kann ferner ausgebildet sein, um Beginn, Dauer und Ende der genannten Zeitintervalle anhand vorbestimmter Bedingungen zu bestimmen.

Die Schreibeinrichtung 48 kann so ausgebildet sein, dass sie nur schreibende aber keine lesende Operationen an der Speichereinrichtung 50 vornimmt. Auch mit dieser Maßnahme kann verhindert werden, dass Daten von der Speichereinrichtung 50 das medizinische Netzwerk 10 stören.

Die Speichereinrichtung 50 umfasst einen nicht-flüchtigen Speicher 52 und einen flüchtigen Speicher 54. Der nicht-flüchtige Speicher 52 umfasst beispielsweise eine Festplatte oder ein anderes nicht-flüchtiges magnetisches oder optisches Speichermedium. Der flüchtige Speicher 54 umfasst beispielsweise einen RAM oder einen anderen Halbleiterspeicher.

Die Schreibeinrichtung 48 und die Speichereinrichtung 50 sind insbesondere ausgebildet, um aktuelle Daten im flüchtigen Speicher 54 und historische Daten im nicht-flüchtigen Speicher 52 der Speichereinrichtung 50 zu schreiben.

Der nicht-medizinische Bereich 60 der Brückenvorrichtung 30 umfasst eine Leseeinrichtung 62. Ein Eingang der Leseeinrichtung 62 ist mit einem Ausgang der Speichereinrichtung 50 gekoppelt. Um anzudeuten, dass die Leseeinrichtung 62 zum Auslesen von Daten aus der Speichereinrichtung 50 an die Speichereinrichtung 50 in einem Lesebefehl übermitteln muss, welche Daten auszulesen sind, sind zwischen der Leseeinrichtung 62 und der Speichereinrichtung 50 zwei Pfeile in entgegengesetzter Richtung gezeigt. Die Leseeinrichtung 62 kann ausgebildet sein, um keine anderen Zugriffe auf die Speichereinrichtung 50 als Lesezugriffe, insbesondere keine schreibenden Zugriffe, auszuführen oder zuzulassen. Auch mit dieser Maßnahme kann verhindert werden, dass Daten von der Speichereinrichtung 50 das medizinische Netzwerk 10 stören. Die Leseeinrichtung 62 kann ferner ausgebildet sein, um von der Speichereinrichtung 50 gelesene Daten aufzubereiten, beispielsweise miteinander logisch oder algebraisch zu verknüpfen.

Der nicht-medizinische Bereich 60 der Brückenvorrichtung 30 umfasst ferner eine Authentifizierungseinrichtung 64 und einen Datenausgang 66 zur Kopplung der Brückenvorrichtung 30 mit dem nicht-medizinischen Netzwerk 20. Die Authentifizierungseinrichtung ist mit der Leseeinrichtung 62 und dem Datenausgang 66 gekoppelt. Die Authentifizierungseinrichtung 64 ist ausgebildet, um Zugriffe aus dem nicht-medizinischen Netzwerk 20 auf die Brückenvorrichtung 30 und insbesondere auf die Speichereinrichtung 50 hinsichtlich der Identität und/oder der Berechtigung des zugreifenden Benutzers zu prüfen und die Identität und die Berechtigung zu verifizieren. Nur Zugriffe von Benutzern aus dem nichtmedizinischen Netzwerk 20, deren Berechtigung verifiziert ist, werden an die Leseeinrichtung 62 weitergegeben.

Die Authentifizierungseinrichtung 64 kann ferner ausgebildet sein, um jeden Zugriff aus dem nicht-medizinischen Netzwerk 20 über den Datenausgang 66 auf die Brückenvorrichtung 30 daraufhin zu prüfen, ob der Zugriff ein Lesezugriff bzw. eine Leseanweisung bzw. ein Lesebefehl ist und jeden anderen Zugriff zu verwerfen bzw. zu ignorieren. Durch diese Ausbildung der Authentifizierungseinrichtung 64 können eine gewollte oder ungewollte Störung der Brückenvorrichtung 30 und eine Störung des medizinischen Netzwerks 10 über die Brückenvorrichtung 30 aus dem nicht-medizinischen Netzwerk 20 verhindert werden.

Der Dateneingang 42 und die Datenempfangseinrichtung 44 oder der Dateneingang 42, die Datenempfangseinrichtung 44 und die Schreibeinrichtung 48 bilden eine erste Schnittstelleneinrichtung zur Kopplung der Speichereinrichtung 50 mit dem medizinischen Netzwerk 10. Die Schreibeinrichtung 48 oder ein Teil der Schreibeinrichtung 48, die Speichereinrichtung 50 und die Leseeinrichtung 62 oder ein Teil der Leseeinrichtung 62 bilden ein Datenbanksystem oder sind Bestandteile eines Datenbanksystems. Alternativ bildet die Speichereinrichtung 50 ein Datenbanksystem oder die Speichereinrichtung 50 umfasst ein Datenbanksystem. Die Authentifizierungseinrichtung 64 und der Datenausgang 66 oder die Leseeinrichtung 62, die Authentifizierungseinrichtung 64 und der Datenausgang 66 bilden eine zweite Schnittstelleneinrichtung zur Kopplung der Speichereinrichtung 50 mit dem nicht-medizinischen Netzwerk 20.

Insgesamt ist die Brückenvorrichtung 30 ausgebildet, um über das medizinische Netzwerk 10 und den Dateneingang 42 Daten von Geräten 14 zu empfangen und, als aktuelle Daten und als (optional wie oben beschrieben aufbereitete) historische Daten in der Speichereinrichtung 50 zu schreiben, und um über ein nicht-medizinisches Netzwerk 30 und einen Datenausgang 66 Anfragen von Geräten 24 zu empfangen und durch Lesen von Daten aus der Speichereinrichtung 50 zu beantworten. Dazu stellt der nicht-medizinische Bereich 60 der Brückenvorrichtung beispielsweise Web-basierte Dienstleistungen bzw. Services zur Kommunikation zur Verfügung, die vom nicht-medizinischen Netzwerk aus angesprochen und nach Authentifizierung genutzt werden können.

Die zweite Schnittstelleneinrichtung, insbesondere die Authentifizierungseinrichtung 64 und die Leseeinrichtung 62 können ausgebildet sein, um ein Subscribing und/oder ein Polling von in der Speichereinrichtung 50 abgelegten Daten vom nicht-medizinischen Netzwerk 20 aus zuzulassen.

Figur 2 zeigt eine schematische Darstellung eines medizinischen Netzwerks 10 und eines nicht-medizinischen Netzwerks 20, die über eine weitere Brückenvorrichtung 30 miteinander gekoppelt sind. Die in Figur 2 gezeigte Brückenvorrichtung 30 unterscheidet sich von der oben anhand der Figur 1 dargestellten Brückenvorrichtung dadurch, dass zwischen dem Dateneingang 42 und der Speichereinrichtung 50 der Brückenvorrichtung 30 eine Einrichtung 46 zur Kopplung in nur einer Richtung angeordnet ist, die physikalisch eine Datenübertragung nur in einer Richtung zulässt. Die Einrichtung 46 erlaubt insbesondere eine Datenübertragung vom Dateneingang 42 zur Speichereinrichtung 50, nicht aber umgekehrt.

Die Einrichtung ist beispielhaft als Einweg-Optokoppler 46 dargestellt, dessen Eingang mit dem Ausgang der Datenempfangseinrichtung 44 und dessen Ausgang mit dem Eingang der Schreibeinrichtung 48 gekoppelt ist. Eine Einrichtung, die physikalisch eine Datenübertragung nur in einer Richtung zulässt, kann auch an einer anderen Stelle zwischen dem Dateneingang 42 und der Speichereinrichtung 50 angeordnet sein. Beispielsweise kann die Einrichtung 46 abweichend von der Darstellung in Figur 2 zwischen dem Dateneingang 42 und der Datenempfangseinrichtung 44 angeordnet sein. Die Einrichtung 46 verhindert mit absoluter Sicherheit eine beabsichtigte oder unbeabsichtigte Störung des medizinischen Netzwerks 10 aus dem nicht-medizinischen Netzwerk 20. Die Einrichtung 46 kann Bestandteil der zweiten Schnittstelleneinrichtung sein.

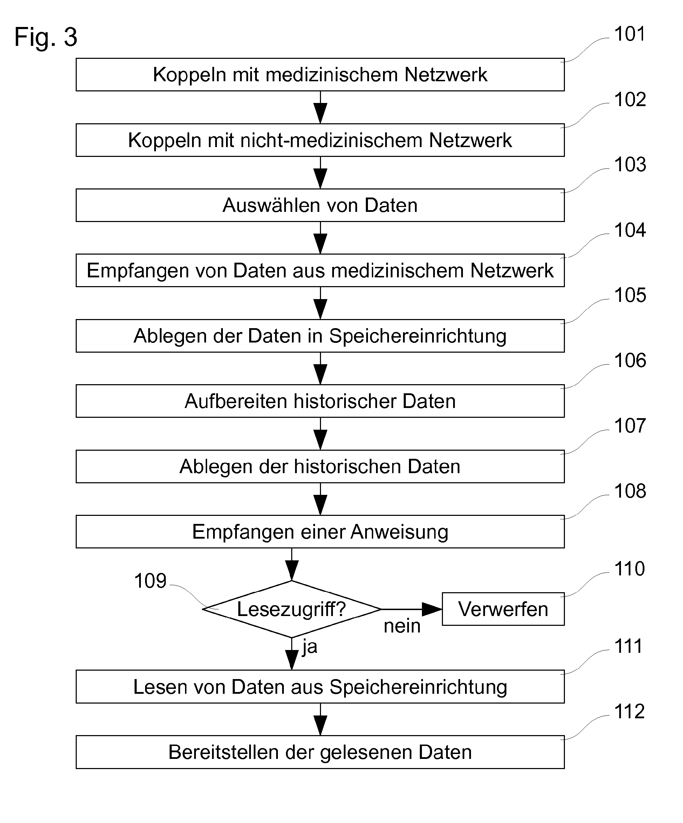

Figur 3 zeigt ein schematisches Flussdiagramm eines Verfahrens zum Bereitstellen von Daten aus einem medizinischen Netzwerk in einem nicht-medizinischen Netzwerk. Obwohl das Verfahren auch mit Vorrichtungen durchführbar ist, die sich von den oben anhand der Figuren 1 und 2 dargestellten Vorrichtungen unterscheiden, werden nachfolgend beispielhaft Bezugszeichen aus den Figuren 1 und 2 verwendet, um ein Verständnis zu erleichtern.

Bei einem ersten Schritt 101 wird eine Brückenvorrichtung 30 über eine erste Schnittstelleneinrichtung 42, 44, 46, 48 mit einem medizinischen Netzwerk 10 gekoppelt. Bei einem zweiten Schritt 102 wird die Brückenvorrichtung 30 über eine zweite Schnittstelleneinrichtung 62, 64, 66 der Brückenvorrichtung 30 mit einem nicht-medizinischen Netzwerk 20 gekoppelt.

Bei einem optionalen dritten Schritt 103 werden Daten aus dem medizinischen Netzwerk 10 bzw. von Geräten 14 aus dem medizinischen Netzwerk 10, die in einer Speichereinrichtung 50 der Brückenvorrichtung 30 zu speichern sind, ausgewählt. Dies erfolgt insbesondere mittels einer Benutzerschnittstelle, die über das medizinische Netzwerk 10 oder direkt mit der Brückenvorrichtung 30 bzw. deren medizinischem Bereich 40 gekoppelt ist. Insofern der medizinische Bereich 40 der Brückenvorrichtung 30 als Teil des medizinischen Netzwerks 10 anzusehen ist, ist auch die Benutzerschnittstelle in beiden Fällen Teil bzw. Teilnehmer des medizinischen Netzwerks 10.

Bei einem vierten Schritt 104 empfängt die Brückenvorrichtung 30 über die erste Schnittstelleneinrichtung 42, 44, 46, 48 Daten aus dem medizinischen Netzwerk 10, insbesondere von einem oder mehreren Geräten 14 im medizinischen Netzwerk 10. Die Daten umfassen insbesondere die Werte von Parametern die von den Geräten 14 eingestellt, gesteuert oder gemessen werden. Bei einem fünften Schritt 105 werden die beim vierten Schritt 14 empfangenen Daten in der Speichereinrichtung 50 der Brückenvorrichtung 30 abgelegt, insbesondere in einem flüchtigen Speicher 54 der Speichereinrichtung 50.

Bei einem sechsten Schritt 106 werden die beim vierten Schritt 104 empfangenen Daten, insbesondere zusammen mit bereits früher empfangenen Daten, zu historischen Daten aufbereitet. Dabei werden für einen Parameter beispielsweise ein Mittelwert, ein Minimalwert, ein Maximalwert innerhalb eines Zeitintervalls und ein Zeitintegralwert von einem vorbestimmten Referenz-Zeitpunkt in der Vergangenheit bis zum Ende des Zeitintervalls berechnet. Bei einem siebten Schritt 107 werden die beim sechsten Schritt 106 aufbereiteten historischen Daten in der Speichereinrichtung 50 abgelegt, insbesondere in einem nicht-flüchtigen Speicher 52 der Speichereinrichtung 50.

Sowohl die aktuellen Daten als auch die historischen Daten können vor dem Ablegen bzw. Schreiben in der Speichereinrichtung 50 mit einem Zeitstempel versehen werden. Mit den historischen Daten werden insbesondere Anfang und Ende des jeweiligen Zeitintervalls oder ein Bezugspunkt innerhalb des Zeitintervalls (beispielsweise Anfang oder Ende des Zeitintervalls) und die Länge des Zeitintervalls in der Speichereinrichtung 50 abgelegt. Alle Daten können zusammen mit zugeordneten Zeitstempeln ohne die oben beschriebene Aufbereitung als historische Daten in der Speichereinrichtung 50 abgelegt werden.

Der vierte Schritt 104, der fünfte Schritt 105, der sechste Schritt 106 und der siebte Schritt 107 werden insbesondere mit einer vorbestimmten Periode bzw. mit einer vorbestimmten Frequenz wiederholt. Alternativ werden diese Schritte abhängig von vorbestimmten Bedingungen bzw. gesteuert durch das Eintreten vorbestimmter Ereignisse ausgeführt. Der vierte Schritt 104, der fünfte Schritt 105, der sechste Schritt 106 und der siebte Schritt 107 können für jeden Parameter unabhängig oder für mehrere oder alle Parameter synchron ausgeführt werden.

Bei einem achten Schritt 108 empfängt die Brückenvorrichtung 30 über den Datenausgang 66 eine Anweisung bzw. einen Befehl. Bei einem neunten Schritt 109 wird geprüft, ob es sich bei der beim achten Schritt 108 empfangenen Anweisung um einen Lesezugriff handelt. Wenn es sich nicht um einen Lesezugriff handelt, und insbesondere wenn es sich um einen Schreibzugriff handelt, wird die Anweisung bei einem zehnten Schritt 110 verworfen bzw. ignoriert.

Wenn die beim achten Schritt 108 empfangene Anweisung ein Lesezugriff ist, werden die dem Lesezugriff entsprechenden Daten bei einem elften Schritt 111 aus der Speichereinrichtung 50 gelesen und bei einem zwölften Schritt 112 am Datenausgang 66 im nichtmedizinischen Netzwerk bereitgestellt.

Bei dem anhand der Figur 3 dargestellten Verfahren kann eine Kommunikation zwischen einem Gerät 24 bzw. einem Nutzer bzw. einem Client aus dem nicht-medizinischen Netzwerk 20 einerseits und der Brückenvorrichtung 30 andererseits wie folgt ablaufen. Vor dem achten Schritt 108 meldet sich das Gerät 24 mittels einer Client-Software bzw. einer Klientensoftware bei der Brückenvorrichtung 30 an und wird optional durch die Authentifizierungseinrichtung 64 authentifiziert. Die Brückenvorrichtung 30 antwortet mit einem Token, der bei der nachfolgenden Kommunikation von der Client-Software des Geräts 24 verwendet wird. Die Brückenvorrichtung 30 übermittelt Information über die in der Speichereinrichtung 50 abgelegten und damit für das Gerät 24 verfügbaren Daten an das Gerät 24.

Das Gerät 24 bzw. sein Benutzer kann über die Client-Software Daten aus den verfügbaren Daten auswählen. Diese Daten werden einmalig (beispielsweise mittels Polling) von der Brückenvorrichtung abgerufen oder abonniert (beispielsweise mittels eines dem oben dargestellten Subscribing ähnlichen Verfahrens). Die verfügbaren Daten umfassen dabei sowohl aktuelle Daten als auch historische Daten. Wenn statische Daten wie Mittelwert, Minimalwert oder Maximalwert innerhalb eines mehrere Zeitintervalle umfassenden Zeitraums abgefragt werden, können diese von der Brückenvorrichtung oder von der Client-Software aus den entsprechenden Daten der einzelnen Zeitintervalle innerhalb des Zeitraums berechnet werden.

Ein Beispiel eines Geräts 24 im nicht-medizinischen Netzwerk 20 ist ein sogenanntes Dashboard, das innerhalb eines Operationssaals oder auch außerhalb desselben angeordnet sein kann, beispielsweise in einem Hörsaal oder in einem Arbeitszimmer von medizinischem Personal. Auf dem Dashboard können alle für eine Beobachtung oder Überwachung einer Operation wichtigen oder relevanten Daten dargestellt werden. Diese wichtigen bzw. relevanten Daten umfassen beispielsweise Patientendaten, Anästhesiedaten, aktuelle Daten von Geräten 14 im medizinischen Netzwerk 10.

### Bezugszeichen

- 10: medizinisches Netzwerk
- 12: Knoten im medizinischen Netzwerk 10
- 14: Gerät im medizinischen Netzwerk 10
- 20: nicht-medizinisches Netzwerk
- 24: Gerät im nicht-medizinischen Netzwerk 20
- 30: Brückenvorrichtung zur Kopplung des medizinischen Netzwerks 10 mit dem nichtmedizinischen Netzwerk 20
- 40: medizinischer Bereich der Brückenvorrichtung 30
- 42: Dateneingang zur Kopplung mit dem medizinischen Netzwerk 10
- 44: Datensammeleinrichtung im medizinischen Bereich 34 der Brückenvorrichtung 30
- 46: Einrichtung zur Kopplung in einer Richtung
- 48: Schreibeinrichtung
- 50: Speichereinrichtung
- 52: nicht-flüchtiger Speicher der Speichereinrichtung 50
- 54: flüchtiger Speicher der Speichereinrichtung 50
- 60: nicht-medizinischer Bereich der Brückenvorrichtung 30
- 62: Leseeinrichtung im nicht-medizinischen Bereich 50 der Monitorvorrichtung 30
- 64: Authentifizierungseinrichtung
- 66: Datenausgang zur Kopplung mit dem nicht-medizinischen Netzwerk 20

## Patentansprüche

1. **Brückenvorrichtung** (30) zur Kopplung eines medizinischen Netzwerks (10) mit einem nicht-medizinischen Netzwerk (20), mit:
einer **Speichereinrichtung** (50) zum Speichern von Daten aus dem medizinischen Netzwerk (10);
einer **ersten Schnittstelleneinrichtung** (42, 44, 48) zur Kopplung der Speichereinrichtung (50) mit dem medizinischen Netzwerk (10);
einer **zweiten Schnittstelleneinrichtung** (62, 64, 66) zur Kopplung der Speichereinrichtung (50) mit dem nicht-medizinischen Netzwerk (20);
wobei die Brückenvorrichtung (30) zumindest entweder ausgebildet ist, um über die erste Schnittstelleneinrichtung (42, 44, 48) **ausschließlich schreibende** Zugriffe auf die Speichereinrichtung auszuführen (50), oder ausgebildet ist, um über die zweite Schnittstelleneinrichtung (62, 64, 66) **ausschließlich lesende** Zugriffe auf die Speichereinrichtung (50) auszuführen.

2. Brückenvorrichtung (30) nach dem vorangehenden Anspruch, bei der zumindest entweder die erste Schnittstelleneinrichtung (42, 44, 48) **ausschließlich für schreibende** Zugriffe auf die Speichereinrichtung (50) ausgebildet ist oder die zweite Schnittstelleneinrichtung (62, 64, 66) **ausschließlich für lesende** Zugriffe auf die Speichereinrichtung (50) ausgebildet ist.

3. Brückenvorrichtung (30) nach einem der vorangehenden Ansprüche, bei der die erste Schnittstelleneinrichtung umfasst:
eine **Schreibeinrichtung** (48) zum Schreiben von Daten aus dem medizinischen Netzwerk (10) in die Speichereinrichtung (50);
einen **Dateneingang** (42) zur Kopplung der Schreibeinrichtung (48) mit dem medizinischen Netzwerk (10).

4. Brückenvorrichtung (30) nach einem der vorangehenden Ansprüche, bei der die zweite Schnittstelleneinrichtung umfasst:
eine Leseeinrichtung (62) zum Lesen von Daten aus der Speichereinrichtung (50);
einen **Datenausgang** (66) zur Kopplung der Leseeinrichtung mit dem nicht-medizinischen Netzwerk (20).

5. Brückenvorrichtung (30) nach einem der vorangehenden Ansprüche, wobei die Brückenvorrichtung (30) ausgebildet ist, um in der Speichereinrichtung (50) **aktuelle Daten** und **historische Daten** zu speichern und über die zweite Schnittstelleneinrichtung (62, 64, 66) an das nicht-medizinische Netzwerk (20) auszugeben.

6. Brückenvorrichtung (30) nach einem der vorangehenden Ansprüche, ferner mit:
einer Einrichtung (48) zur **Aufbereitung** von über die erste Schnittstelleneinrichtung empfangenen Daten, wobei die Einrichtung (48) zumindest entweder zur Bestimmung eines Mittelwerts oder zur Bestimmung eines Zeitintegrals oder zur Bestimmung eines Extremwerts innerhalb eines vorgegebenen Zeitintervalls ausgebildet ist.

7. Brückenvorrichtung (30) nach einem der vorangehenden Ansprüche, bei der die Speichereinrichtung (50) einen **flüchtigen** Speicher (54) und einen **nicht-flüchtigen** Speicher (52) umfasst.

8. Brückenvorrichtung (30) nach einem der vorangehenden Ansprüche, wobei die Brückenvorrichtung (30) ausgebildet ist, um über die erste Schnittstelleneinrichtung (42, 44, 48) empfangenen Daten mit einem **Zeitstempel** zu versehen.

9. Brückenvorrichtung (30) nach einem der vorangehenden Ansprüche, wobei die Brückenvorrichtung (30) ausgebildet ist, um Daten einer Operation zu archivieren oder zu **dokumentieren.**

10. Brückenvorrichtung (30) nach einem der vorangehenden Ansprüche, wobei die Brückenvorrichtung (30) ausgebildet ist, um zumindest entweder
keine Fehlermeldung in das medizinische Netzwerk (10) abzugeben, oder
jede Fehlermeldung aus einer vorbestimmten Gruppe von Fehlermeldungen an Empfänger im medizinischen Netzwerk (10) zu unterdrücken.

11. **Verfahren zum Bereitstellen von Daten aus einem medizinischen Netzwerk** (10) in einem nicht-medizinischen Netzwerk (20), mit folgenden Schritten:
Koppeln (101) einer **Brückenvorrichtung** (30) über eine **erste Schnittstelleneinrichtung** (42, 44, 48) der Brückenvorrichtung (30) mit dem medizinischen Netzwerk (10);
Koppeln (102) der Brückenvorrichtung (30) über eine **zweite Schnittstelleneinrichtung** (62, 64, 66) der Brückenvorrichtung (30) mit dem nicht-medizinischen Netzwerk (20);
**Empfangen** (104) von Daten aus dem medizinischen Netzwerk (10) über die erste Schnittstelleneinrichtung (42, 44, 48);
**Schreiben** (105) der empfangenen Daten an einen Speicherort einer Speichereinrichtung (50) der Brückenvorrichtung (30);
Lesen (111) von Daten von der Speichereinrichtung (50);
**Bereitstellen** (112) der von der Speichereinrichtung (50) gelesenen Daten an der zweien Schnittstelle (62, 64, 66).

12. Verfahren nach dem vorangehenden Anspruch, ferner mit zumindest einem der folgenden Schritte:
**Auswählen** (103) von Daten aus dem medizinischen Netzwerk (10), die in der Speichereinrichtung (50) zu speichern sind, an einer **Benutzerschnittstelle im medizinischen Netzwerk** (10);
Empfangen (108) einer Leseanweisung über die zweite Schnittstelleneinrichtung (62, 64, 66);
**Verwerfen** einer über die erste Schnittstelleneinrichtung (42, 44, 48) empfangenen Leseanweisung;
**Verwerfen** (110) einer über die zweite Schnittstelleneinrichtung (62, 64, 66) empfangenen Schreibanweisung;
**Prüfen** (109) einer über die zweite Schnittstelleneinrichtung (62, 64, 66) empfangenen Anweisung und **Verwerfen** (110) der Anweisung, wenn diese keine Leseanweisung ist;
Aufbereiten (106) **historischer Daten;**
Schreiben (107) **historischer Daten** in die Speichereinrichtung (50).

13. **Computer-Programm** mit Programmcode zur Durchführung oder Steuerung eines Verfahrens nach einem der vorangehenden Ansprüche, wenn das Computer-Programm auf einem Computer oder einem Prozessor abläuft.
